# EUROPÄISCHE PATENTSCHRIFT

(11) **EP 0 503 419 B1**
(45) Veröffentlichungstag und Bekanntmachung des Hinweises auf die Patenterteilung: **09.07.1997**
(21) Anmeldenummer: 92103534.1
(22) Anmeldetag: 02.03.1992
(51) Int. Cl.: C12P 7/42

(54) **Verfahren zur Herstellung von MA**
Process for production of MA
Procédé de production de MA

(30) Priorität: 08.03.1991 DE 4107460
(43) Veröffentlichungstag der Anmeldung: 16.09.1992
(73) Patentinhaber: HOECHST AKTIENGESELLSCHAFT, 65926 Frankfurt am Main (DE)
(72) Erfinder: Aretz, Werner, Dr., W-6240 Königstein/Taunus (DE); Ehlers, Eberhard, Dr., W-6238 Hofheim am Taunus (DE); Hedtmann, Udo, Dr., W-6230 Frankfurt am Main 80 (DE)

(56) Entgegenhaltungen:
- EP-A- 0 355 679
- US-A- 3 660 569

## Beschreibung

Moenomycin A ist die Hauptkomponente des in der Tierernährung verwendeten Flavomycin®. Wie andere bekannte Phosphoglycolipidantibiotika hemmt es die Biosynthese des Peptidoglycangerüsts der bakteriellen Zellwand. Genauere Untersuchungen zeigten, daß die Transglycosylierungsreaktion des Penicillin-Bindeproteins 1b von E. coli durch diese Stoffe inhibiert wird [Huber G., Antibiotics, V-1, S. 135 - 153, (1979)]. Versuche, Phosphoglykolipidantibiotika enzymatisch bzw. mikrobiell gezielt abzubauen, scheiterten anfangs.

In der europäischen Anmeldung EP 0 355 679 ist ein Verfahren zum Abbau der Moenomycine (= Phosphoglycolipidantibiotikum) zu MA, MB und MC, katalysiert durch die Enzyme Moenomycinase und MBase aus Bacillus sp. DSM 4675, beschrieben.

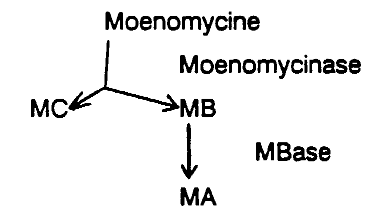

Zu den Antibiotika der Moenomycingruppe gehören beispielsweise das Pholipomycin¹⁾, die Prasinomycine²⁾, die Diumycine (Macarbomycine)³ Esanchomycin, Prenomycin und Teichimycin, sowie andere strukturell verwandte Substanzen, die eine entsprechende funktionalisierte Phosphoglycerinsäure aufweisen
[1) S. Takahashi et al., Tetrahedron Lett. 1983, 499
2) F.L. Weisenborn et al., Nature 213,1092 (1967)
3) S. Takahashi et al., J. Antibiot. 26, 542 (1973)].

In EP 0 355 679 werden außerdem die aerobe Fermentation von Bacillus spec. DSM 4675, die beim Abbau der Moenomycine entstehenden Spaltprodukte, die den Abbau katalysierenden Enzyme und die Verwendung der Abbauprodukte als Synthesebausteine zur Herstellung von Transglycosylase-Inhibitoren (MA) bzw. als antibiotisch wirksamen Stoff (MB) dargestellt.

Das Verfahren in o.g. Anmeldung ergibt eine 1 %ige Ausbeute von MA, da es auf das biologisch aktive, d.h. antibiotisch wirkende MB ausgerichtet ist.

Es besteht jedoch ein deutlicher Bedarf, Verfahren zur Herstellung von MA zu optimieren, da MA einen wertvollen Baustein für neue MA-Analoge, d.h. für neue Transglycosylase-lnhibitoren darstellt.

Die Erfindung betrifft somit:

1. Ein Verfahren zur Herstellung von MA der Formel I 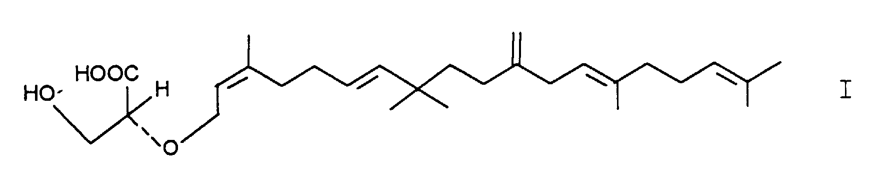 durch enzymatischen Abbau von Phosphoglycolipiden, das dadurch gekennzeichnet ist, daß die enzymatische Katalyse in einem Glycin-NaOH-Puffers erfolgt.

Vorzugsweise werden für die enzymatische Katalyse Enzyme aus Bacillus sp. DSM 4675 eingesetzt. Diese können besonders bevorzugt auch in Form der lyophilisierten Zellen von Bacillus sp. DSM 4675 verwendet werden.

Vorzugsweise werden die Zellen von Bacillus sp. DSM 4675 zunächst in einem Anzuchtmedium angezüchtet, welches für Bacillus sp. DSM 4675 bezüglich Moenomycinase- und MBase-Titer durch Zusatz von Phosphat optimiert wird.

Die Erfindung wird im folgenden detailliert beschrieben, insbesondere in den bevorzugten Ausführungsformen. Ferner wird sie in den Ansprüchen definiert.

Prozentangaben beziehen sich, wenn nichts anderes angegeben ist, auf das Gewicht.

Bacillus sp. wurde mit der Nummer DSM 4675 unter den Bedingungen des Budapester Vertrags bei der Deutschen Sammlung von Mikroorganismen und Zellkulturen GmbH in Braunschweig, Deutschland, am 23.6.1988 hinterlegt.

Das Wachstum des Mikroorganismus Bacillus sp. DSM 4675 und die Bildung der für die erfindungsgemäßen Abbaureaktionen notwendigen Enzyme ist besonders gut in einem Nährmedium mit den Hauptbestandteilen: Citronensäure, Natrium-Gluconat, Glycerin, Pepton, Phosphat und einer Vitaminlösung. Die Konzentration des Phophates, z.B. Kaliumphosphat, liegt vorzugsweise bei 50-100 mM. Das Nährmedium kann jedoch auch ohne Phosphat oder mit Phosphat in jeder beliebigen, physiologischen Konzentration eingesetzt werden. Der Anteil der Gluconsäure bzw. deren Salz liegt bei 1-2 %, vorzugsweise 2 %.

Die Fermentation erfolgt aerob, also beispielsweise submers unter Schütteln oder Rühren in Schüttelkolben oder Fermentern, gegebenenfalls unter Einführen von Luft oder Sauerstoff. Die Fermentation kann in einem Temperaturbereich von etwa Raumtemperatur bis 50 °C, vorzugsweise bei etwa 35 bis 37 °C, durchgeführt werden. Die Kulturzeit beträgt im allgemeinen 8 bis 48 Stunden, vorzugsweise 16-18 Stunden.

Wie in EP 0 355 679 beschrieben, ist es bei Verwendung von Bacillus-Zellen vorteilhaft, diese zu permeabilisieren, beispielsweise mit Cetyltrimethylammoniumsalzen, oder zu lyophilisieren. Es ist ebenfalls möglich, mit Proteinisolaten aus den Bacillus-Zellen bzw. mit Enzym-Extrakten, die durch Aussalzen oder Chromatographie partiell angereichert wurden, oder natürlich mit dem gereinigten Enzym zu arbeiten. Ferner ist es möglich, das Enzym in freier oder immobilisierter Form einzusetzen.

Als Enzymquelle für die enzymatische Spaltung der Moenomycine zu MA werden im erfindungsgemäßen Verfahren vorzugsweise lyophilisierte Zellen eingesetzt.

Aus dem Schema auf Seite 1 geht hervor, daß zur Herstellung von MA zwei Enzyme notwendig sind. Zur Spaltung der phosphoglykosidischen Bindung des Moenomycins A wird ein Enzym benötigt, das von den Erfindern Moenomycinase genannt wurde. Moenomycinase ist mit der Cytoplasmamembran von Bacillus sp. DSM 4675 assoziiert und kann nach an sich bekannten Methoden zur Enzymisolierung aus dem Mikroorganismus gewonnen werden.

MBase kann ebenfalls nach bekannten Methoden aus dem Mikroorganismus isoliert werden. Beispielsweise werden die Zellen mit Ultraschall aufgeschlossen und der resultierende Rohextrakt entweder durch Ammoniumsulfatfraktionierung (25-55 % Sättigung) oder Ultrazentrifugation weiter angereichert. Danach erfolgt Dialyse. Abschließend werden die Moenomycinase und MBase chromatographisch getrennt.

Bei dem erfindungsgemäßen Verfahren erfolgt die enzymatische Spaltung, d.h. die Umsetzung der Moenomycine zu MA, vorzugsweise in einem Ansatz (Beispiel 2).

Die Spaltung der Moenomycine wird mit lyophilisierten Zellen oder Enzymisolaten, vorzugsweise jedoch mit lyophilisierten Zellen, durchgeführt.

Die Reaktion erfolgt im Glycin-NaOH-Puffer. Der pH-Wert des Puffers liegt vorzugsweise bei pH 8,0-8,5, ansonsten im Bereich von pH 7,5-10. Die Umsetzung erfolgt bei 34 - 39 °C, vorzugsweise bei 37 °C. Der pH-Wert der Enzymreaktion liegt im Bereich von pH 7,0-9,0, vorzugsweise 7,8. Die Umsetzungszeit beträgt im allgemeinen 5-48 Stunden, bevorzugt ca. 24 Stunden. Die Substratkonzentration sollte im Bereich von 0,1 bis 5 %, bevorzugt 1 bis 2 % liegen.

Bei höheren oder niedrigeren Temperaturen bzw. pH-Werten als angegeben kann die Reaktion ebenfalls noch durchgeführt werden. Jedoch ist die Enzymaktivität dann geringer.

Für den Abbau von MB zu MA kann außer der MBase, wie in EP 0 355 679 beschrieben, auch Phosphatase eingesetzt werden. Vorzugsweise werden die saure Phosphatase aus Kartoffeln und die alkalische Phosphatase aus Kälberdarm verwendet. Die Enzyme sind käuflich erhältlich (Sigma).
Beide Enzyme können in immobilisierter und nicht-immobilisierter Form eingesetzt werden.

Das durch die Spaltungsreaktion gewonnene MA der Formel I wird anschließend isoliert und aufgereinigt. Dies geschieht durch Extraktion des Filtrats der Biomasse bzw. der Biomasse selbst mit organischen Lösemitteln. Als Lösungsmittel wird vorzugsweise Aceton in einem Volumenverhältnis von 0,2-1, vorzugsweise 0,3, eingesetzt. Die chromatographische Reinigung erfolgt durch Verwendung eines Petrolether-Aceton- oder Petrolether-Ethylacetat-Gemisches als Waschflüssigkeit. Als MA-Eluens wird Methanol eingesetzt.

Das enstandene Reaktionsprodukt MA kann als Synthesebaustein für Transglycosylase-Inhibitoren verwendet werden.

Anhand von Beispielen wird die Erfindung weitergehend beschrieben.

### Beispiel 1

### Stammhaltung von Bacillus sp. DSM 4675

Stammhaltung und Anzucht der Vorkultur sind in der Europäischen Patentanmeldung 0 355 679 (Beispiele 1 und 2) beschrieben.

a) Als Hauptkulturstufe dient ein 12 l-Laborfermenter, gefüllt mit 9 l Medium der folgenden Zusammensetzung:

| | |
|---|---|
| Pepton | 12,5 g/l |
| Glycerin | 20,0 g/l |
| Citrat | 2,0 g/l |
| Na-Gluconat | 10,0 g/l |
| K₂HPO₄ | 10,0 g/l |
| MgSO₄ x 7H₂O | 0,5 g/l |
| FeCl₃ x 6H₂O | 0,04 g/l |
| Vitaminlösung | 1 ml |

### Vitaminlösung:

| | |
|---|---|
| Nicotinsäure | 0,35 g/l |
| Thiamin HCl | 0,30 g/l |
| D-Biotin | 0,01 g/l |
| p-Aminobenzoesäure | 0,20 g/l |
| Pyridoxal HCl | 0,10 g/l |
| Ca-Pantothenat | 0,10 g/l |
| Vitamin B12 | 0,05 g/l |

Dieser wird mit 500 ml Vorkultur 16-18 Stunden bei 37 °C, 300 Upm und einer Belüftungsrate von 0,5 vvm inkubiert.

Die ausgewachsene Kultur wird abzentrifugiert und anschließend lyophilisiert.

Das neue Medium und die verkürzte Fermentationszeit führen zu einer Verdopplung der Biomasseausbeute. Die Biomasse wird mit Hilfe der Optischen Dichte charakterisiert. Man mißt OD = 7. Außerdem erhält man Zellen, die Moenomycine mit hoher Ausbeute zu MA abbauen.

In einem Testansatz mit 100 µl Rohextrakt, 12 mg Moenomycin A und 900 µl Kaliumphosphatpuffer (pH 8,0) 50 mM werden innerhalb von 7-24 Stunden bei 37 °C 50 % des eingesetzten Substrates abgebaut. Als Reaktionsprodukte findet man MA, MB und MC.

b) Verwendet man das unter a) beschriebene Medium, jedoch ohne Zusatz von Phosphat, ist die Biomasse deutlich reduziert. Man mißt OD = 3.

### Beispiel 2

### Umsetzung der Moenomycine zu MA (Enzymatische Spaltung)

Es werden lyophilisierte Zellen von Bacillus sp. DSM 4675 zur Umsetzung verwendet.

a) 90-200 g Lyophilisat werden in 9 l Glycin-NaOH-Puffer, 100 mM, pH 8,5 suspendiert und nach Zugabe von 135 g Moenomycin-Gemisch oder nach Zugabe von MB, 1,8 Na-Azid und 214 mg CoCl₂ für 6-48 Stunden bei 37 °C und 190 UpM inkubiert.

Der Reaktionsverlauf, der mittels DC-Analyse verfolgt wird, zeigt, daß bis zu 80 % des Substrates zu MB und MA abgebaut werden. Davon entfallen ca. 10-20% der Spaltprodukte auf MB und ca. 80-90 % auf MA.

Durch diese Reaktionsführung ist eine MA-Produktion im Maßstab möglich.

b) Setzt man einen Puffer identischer Zusammensetzung, jedoch mit pH 9,0 ein, erhält man eine Ausbeute von 60 % MA.

c) Bei Verwendung eines TRIS-HCl-Puffers [100 mM Tris, pH 7,8, ansonsten entspricht die Zusammenseztung dem unter a) angegebenen Puffer] reduziert sich die Ausbeute an MA ebenfalls auf 60 %.

### Beispiel 3

### Enzymatische Spaltung von MB mittels Phosphatasen

Untersucht wurde der Einsatz von saurer Phosphatase aus Kartoffeln und alkalischer Phosphatase aus Kälberdarm zur Herstellung von MA aus MB.

Die saure Phosphatase (10 U/ml Ansatz) setzt bei pH 4,8 und Raumtemperatur MB (5 mg/ml) zu ca. 50 %, die alkalische Phosphatase (50 U/ml) bei pH 8,0 zu über 90 % innerhalb von 144 Stunden zu MA um. Die Umsatzgeschwindigkeit der alkalischen Phosphatase ist bei pH 10,5 (Glycin-NaOH-Puffer, 100 mM) und 37 °C in Gegenwart von 0,1 mM ZnCl₂ und MgCl₂ deutlich erhöht (< 24 Std.).

Eine Verwendung von immobilisierter alkalischer Phosphatase ist ebenfalls möglich. Dabei setzen 35 U/ml unter oben genannten Bedingungen über 90 % des Substrates innerhalb von 28 Std. um.

### Beispiel 4

### Isolierung des Flavomycin-Abbauproduktes MA

Aus der nach der enzymatischen Umsetzung vorliegenden Suspension werden die Feststoffanteile abzentrifugiert.

Die anfallende Biomasse wird mehrmals solange mit dem gleichen Volumen an Aceton bei Raumtemperatur ausgerührt, bis in der organischen Phase dünnschichtchromatographisch kein MA mehr nachzuweisen ist. Die MA-enthaltenden Extrakte werden vereinigt.

Das nach dem Entfernen der Biomasse erhaltene Filtrat wird zunächst einmal mit 1/3 seines Volumens an Ethylacetat extrahiert. Danach wird die wäßrig-organische, kolloidale Lösung solange mit 1/3 ihres Volumens an Aceton extrahiert, bis in der jeweils abtrennbaren organischen Phase mittels DC kein MA mehr nachzuweisen ist.

Die MA-enthaltenden Extrakte werden mit den Aceton-Ausrührungen der Biomasse vereinigt und im Vakuum vom Lösungsmittel befreit.

Aus der verbleibenden wäßrigen Reaktionslösung kann die Flavomycin-Komponente MB durch mehrmaliges Extrahieren mit n-Butanol isoliert werden. Das nach dem Abdestillieren des Lösungsmittels im Vakuum erhaltene rohe MB kann erneut der enzymatischen Reaktion zur Herstellung von MA zugeführt werden.

Im Mittel werden aus ca. 500 g des Flavomycin-A/C-Komplexes ca. 110 g an rohem MA und ca. 85 g an rohem MB nach dem oben beschriebenen Extraktionsverfahren erhalten.

### Beispiel 5

### Chromatographische Reinigung von MA

Das nach dem Evaporieren des Lösungsmittels vorliegende MA wird anschließend säulenchromatographisch an Kieselgel gereinigt.

Hierzu werden ca. 20 g an rohem MA in der minimalsten Mengen eines 1:1-Gemisches an Petroläther/Aceton gelöst und bei 7-10 bar mit einem Fluß von 5 l/h auf eine Stahlsäule mit ca. 2,1 kg Kieselgel 60 (pH = 7,5) als stationäre Phase aufgetragen. Anschließend wird unter den gleichen Bedingungen mit ca. 10 l eines Gemisches aus Petroläther/Aceton (6 : 4) nachgewaschen. Die Waschflüssigkeit wird in einer einzigen Fraktion gesammelt. Danach wird mit ca. 5 l reinem Methanol eluiert.

Das Methanol-Eluat wird jeweils in Fraktionen von 0,1 l gesammelt. Die durch DC detektierten MA-aktiven Reinfraktionen werden vereinigt und im Vakuum vom Lösungsmittel befreit. MA fällt als hellgelbes, hochviskoses Öl an. Randfraktionen können rechromatographiert werden. Aus ca. 20 g rohem MA werden etwa 13 g an Reinsubstanz erhalten.

Zum dünnschichtchromatographischen Nachweise von MA werden Kieselgel-Fertigplatten verwendet. Als Fließmittel dient ein Lösungsmittelgemisch aus Chloroform/Methanol/Essigsäure (80: 10: 1). Die Detektion erfolgt durch Anfärbung der entwickelten Platten mit PMS und ihrem anschließendem Trocknen bei 130 °C. Als Vergleichssubstanzen werden MA, MB und der Flavomycin-A/C-Komplex mitaufgetragen.

## Patentansprüche

1. Verfahren zur Herstellung der Verbindung der Formel I 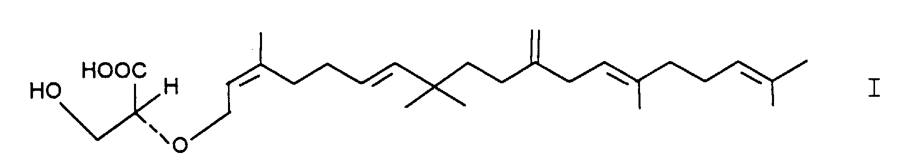 durch enzymatischen Abbau von Phosphoglycolipiden, dadurch gekennzeichnet, daß die enzymatische Katalyse in einem Glycin-NaOH-Puffer erfolgt.

2. Verfahren nach Anspruch 1, dadurch gekennzeichnet, daß der Glycin-NaOH-Puffer den pH-Wert 8,0-8,5 hat.

3. Verfahren nach Anspruch 1 oder 2, dadurch gekennzeichnet, daß für die enzymatische Katalyse Enzyme aus Bacillus sp. DSM 4675 eingesetzt werden.

4. Verfahren nach Anspruch 3, dadurch gekennzeichnet, daß als Enzymquelle lyophilisierte Zellen von Bacillus sp. DSM 4675 eingesetzt werden.

5. Verfahren nach Anspruch 4, dadurch gekennzeichnet, daß die Zellen von Bacillus sp. DSM 4675 zunächst in einem Anzuchtmedium angezüchtet werden, welches für Bacillus sp. DSM 4675 bezüglich Moenomycinase- und MBase-Titer durch Zusatz von Phosphat optimiert wird.

6. Verfahren nach Anspruch 5, dadurch gekennzeichnet, daß dem Anzuchtmedium das Phosphat in einer Konzentration von 50 bis 100 mM zum Puffer zugesetzt wird.

7. Verfahren nach Anspruch 1, dadurch gekennzeichnet, daß für die enzymatische Katalyse eine saure oder alkalische Phosphatase eingesetzt wird.

8. Verfahren nach Anspruch 7, dadurch gekennzeichnet, daß die saure Phosphatase aus der Kartoffel eingesetzt wird.

9. Verfahren nach Anspruch 7, dadurch gekennzeichnet, daß die alkalische Phosphatase aus Kälberdarm eingesetzt wird.

10. Verfahren nach einem der Ansprüche 4 bis 6 , dadurch gekennzeichnet, daß das Filtrat der Biomasse mit Ethylacetat und anschließend mit Aceton extrahiert, sowie die Biomasse selbst mit Aceton ausgerührt wird.

11. Verfahren nach Anspruch 10, dadurch gekennzeichnet, daß das Filtrat der Biomasse und die Biomasse selbst mit Aceton in einem Volumenverhältnis von 1:0,3 versetzt wird.

## Claims

1. A process for the preparation of the compound of the formula I 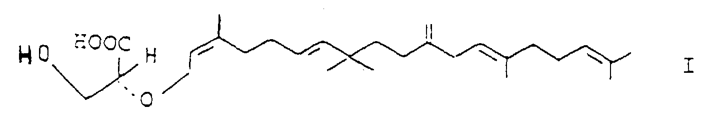 by enzymatic degradation of phosphoglycolipids, wherein the enzymatic catalysis takes place in a glycine/NaOH buffer.

2. The process as claimed in claim 1, wherein the glycine/NaOH buffer has the pH 8.0-8.5.

3. The process as claimed in claim 1 or 2, wherein enzymes from Bacillus sp. DSM 4675 are employed for the enzymatic catalysis.

4. The process as claimed in claim 3, wherein lyophilized cells of Bacillus sp. DSM 4675 are employed as enzymes source.

5. The process as claimed in claim 4, wherein the cells of Bacillus sp. DSM 4675 are initially cultivated in a culture medium which is optimized for Bacillus sp. DSM 4675 with respect to the titers of moenomycinase and MBase by addition of phosphate.

6. The process as claimed in claim 5, wherein the phosphate is added in a concentration of 50 to 100 mM to the buffer to the culture medium.

7. The process as claimed in claim 1, wherein an acid or alkaline phosphatase is employed for the enzymatic catalysis.

8. The process as claimed in claim 7, wherein the acid phosphatase from potato is employed.

9. The process as claimed in claim 7, wherein the alkaline phosphatase from calf intestine is employed.

10. The process as claimed in any of claims 4 to 6, wherein the filtrate of the biomass is extracted with ethyl acetate and then with acetone, and the biomass itself is extracted by stirring with acetone.

11. The process as claimed in claim 10, wherein the filtrate of the biomass and the biomass itself are mixed with acetone in a ratio of 1:0.3 by volume.

## Revendications

1. Procédé de préparation du composé de formule I 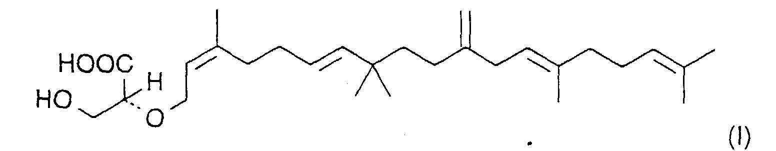 par dégradation enzymatique de phosphoglycolipides, caractérisé en ce que la catalyse enzymatique est effectuée dans un tampon glycine/NaOH.

2. Procédé selon la revendication 1, caractérisé en ce que le tampon glycine/NaOH a un pH de 8,0 à 8,5.

3. Procédé selon la revendication 1 ou 2, caractérisé en ce que l'on utilise des enzymes du *Bacillus sp.* DSM 4675 pour la catalyse enzymatique.

4. Procédé selon la revendication 3, caractérisé en ce que des cellules lyophilisées de *Bacillus sp.* DSM 4675 représentent la source d'enzymes.

5. Procédé selon la revendication 4, caractérisé en ce que les cellules de *Bacillus sp.* DSM 4675 sont d'abord cultivées dans un milieu de culture optimisé pour le *Bacillus sp.* DSM 4675 en ce qui concerne le titre de moénomycinase et de MBase, par ajout de phosphate.

6. Procédé selon la revendication 5, caractérisé en ce que l'on ajoute du phosphate à une concentration de 50 à 100mM par rapport au tampon, au milieu de culture.

7. Procédé selon la revendication 1, caractérisé en ce que l'on utilise une phosphatase acide ou alcaline pour la catalyse enzymatique.

8. Procédé selon la revendication 7, caractérisé en ce que l'on utilise la phosphatase acide de pomme de terre.

9. Procédé selon la revendication 7, caractérisé en ce que l'on utilise la phosphatase alcaline d'intestin de veau.

10. Procédé selon une des revendications 4 à 6, caractérisé en ce que le filtrat de la biomasse est extrait avec de l'acétate d'éthyle puis avec de l'acétone, et la biomasse elle-même est extraite par mélange à de l'acétone.

11. Procédé selon la revendication 10, caractérisé en ce que le filtrat de la biomasse et la biomasse elle-même sont mis en présence d'acétone avec une proportion volumique de 1 pour 0,3.
